# EUROPÄISCHE PATENTSCHRIFT

(11) **EP 2 118 262 B1**
(45) Veröffentlichungstag und Bekanntmachung des Hinweises auf die Patenterteilung: **12.07.2017**
(21) Anmeldenummer: 08707672.5
(22) Anmeldetag: 12.02.2008
(51) Int. Cl.: C12M 1/00, C12M 1/12

(54) **BIOREAKTOR, ANORDNUNG AUS BIOREAKTOREN, VERFAHREN ZU IHRER HERSTELLUNG UND IHRE VERWENDUNG**
BIOREACTOR, ARRANGEMENT OF BIOREACTORS, PROCESS FOR PRODUCTION THEREOF AND USE THEREOF
BIORÉACTEUR, AGENCEMENT DE BIORÉACTEURS, PROCÉDÉ POUR LEUR FABRICATION ET LEUR UTILISATION

(30) Priorität: 16.02.2007 DE 102007007718
(43) Veröffentlichungstag der Anmeldung: 18.11.2009
(73) Patentinhaber: Karlsruher Institut für Technologie, 76131 Karlsruhe (DE)
(72) Erfinder: WEIBEZAHN, Karl-Friedrich, 76297 Stutensee (DE); GOTTWALD, Eric, 76149 Karlsruhe (DE); TRUCKENMÜLLER, Roman, 74223 Flein (DE); GISELBRECHT, Stefan, 76187 Karlsruhe (DE)
(74) Vertreter: Weddigen, Andreas
(86) Internationale Anmeldenummer: PCT/EP2008/001048
(87) Internationale Veröffentlichungsnummer: WO 2008/098733

(56) Entgegenhaltungen:
- EP-A- 0 279 077
- EP-A- 1 431 030
- WO-A-02/057405
- DE-A1- 10 150 269
- DE-U1- 8 908 583
- DE-U1-202006 012 978
- US-A- 4 939 151
- US-A- 5 843 766

## Beschreibung

Die Erfindung betrifft einen Bioreaktor für die Kultivierung von Zellen, insbesondere für ihre Langzeitkultivierung, eine Anordnung aus derartigen Bioreaktoren, Verfahren zu ihrer Herstellung und ihre Verwendung.

Gängige Bioreaktoren sind aus mehreren Teilen zusammengesetzt, wobei die Zellkultursubstrate nicht mit dem Bioreaktor verbunden sind, sondern getrennt eingesetzt werden. Darüber hinaus bestehen diese Teile üblicherweise aus verschiedenen Materialien, die zumeist getrennte Herstellungsverfahren erfordern.

Aus der DE 41 32 379 C2 ist ein Substrat für Zellkulturen bekannt, das aus einem plattenförmigen Körper besteht, der mit einer Vielzahl von durch Stege voneinander getrennten Mikrocontainern zur Aufnahme von Zellen oder Zellaggregaten versehen ist.

Die DE 10 2004 035 267 B3 offenbart einen Formkörper aus einer Folie, in die mindestens eine Hohlstruktur und eine Vielzahl von Poren eingebracht sind, sowie dessen Verwendung zur Kultivierung von Zellen und als Bioreaktor.

Die 20 2006 012 978 U1 beschreibt einen Bioreaktor, der aus einer Basisplatte besteht, die eine Aussparung zur Aufnahme einer Poren aufweisenden Trägerstruktur für Zellkulturen,
eine obere und eine untere transparente Deckelfolie sowie Kanäle zur Versorgung der sich auf der Trägerstruktur befindlichen Zellkulturen aufweist.

Die DE 89 08 583 U1 zeigt einen Träger für die Zellkultur und die dazugehörige Vorrichtung zur Kultur von Zellen auf dem Träger. Insbesondere im Nahrungsmittelbereich, werden thermogeformte Klappschachteln auf vielfältige Art und Weise eingesetzt. Dabei sind zwei Halbschalen, so genannte *Trays,* eine Unter- und eine Oberschale oder eine Unterschale und ein zugehöriger Deckel über ein Foliengelenk miteinander verbunden. Typischerweise werden diese Teile in dieselbe Folie geformt. Beim Foliengelenk wird durch eine geeignete Formgebung ähnlich einer Sicke die definierte Biegung bzw. Knickung entlang einer geraden Linie in der anfänglichen Folienebene angestrebt. Die wiederverschließbare Verbindung der zusammengeklappten Teile wird z.B. dadurch verwirklicht, dass ein umlaufender Steg in der einen Schale in einer dann umlaufenden Nut bzw. Sicke in der anderen klemmt oder dass in einer Unterschale ein in die gleiche Richtung, jedoch flacher ausgeformter Deckel klemmt. Schräge Seitenwände dienen hierbei der Zentrierung als auch der (Selbst-)Hinderung gegen selbsttätiges Wiederöffnen.

Ausgehend hiervon ist es die Aufgabe der vorliegenden Erfindung, einen Bioreaktor vorzuschlagen, der einfach herstellbar und einfach handhabbar ist. Insbesondere soll sich ein solcher Bioreaktor auch als Einmalteil eignen und daher nach Gebrauch nicht gereinigt und sterilisiert werden müssen.

Diese Aufgabe wird im Hinblick auf den Biorektor durch die Merkmale des Anspruchs 1, im Hinblick auf eine Anordnung aus Bioreaktoren durch das Merkmal des Anspruchs 11, im Hinblick auf Verfahren zu ihrer Herstellung durch die Merkmale des Anspruchs 12 und im Hinblick auf deren Verwendung durch den Anspruch 14 gelöst. Die Unteransprüche beschreiben jeweils vorteilhafte Ausgestaltungen der Erfindung.

Ein erfindungsgemäßer Bioreaktor in Form einer Klappschachtel besteht aus einem Gehäuse aus einer unteren Gehäusehälfte und einer oberen Gehäusehälfte, die mittels eines Foliengelenks an jeweils einer Seite miteinander verbunden sind, wobei an mindestens einer Gehäusehälfte Mittel zum Austausch von Fluiden vorgesehen sind, und mindestens einem Trägerelement für mindestens ein Zellkultursubstrat, wobei der Bioreaktor aus einem warmumformbaren thermoplastischen oder thermoelastischen Folienmaterial besteht. In einer bevorzugten Ausgestaltung ist das mindestens eine Trägerelement derart zwischen die untere Gehäusehälfte und die obere Gehäusehälfte eingefügt, dass es im zusammengeklappten Zustand zwischen den beiden Gehäuseteilen liegt.

In einer alternativen Ausgestaltung ist das mindestens eine Trägerelement derart in die untere Gehäusehälfte oder in die obere Gehäusehälfte eingefügt, dass es selbst Bestandteil der betreffenden Gehäusehälfte ist oder fest mit ihr verbunden ist.

In einer besonderen Ausgestaltung umfasst der Bioreaktor zwei Trägerelemente, von denen ein Trägerelement Bestandteil einer der beiden Gehäusehälfte ist, während das andere Trägerelement zwischen den beiden Gehäusehälften eingefügt ist. Diese Ausgestaltung ermöglicht z.B. die Kultivierung von Co-Kulturen.

Vorzugsweise sind die untere Gehäusehälfte und die obere Gehäusehälfte fluiddicht, d.h. flüssigkeits- oder gasdicht, zueinander angeordnet. In einer bevorzugten Ausgestaltung ist hierbei das mindestens eine Trägerelement fluiddicht zu einer der beiden Gehäusehälften oder zwischen beiden Gehäusehälften angeordnet. Hierzu besitzt das mindestens eine Trägerelement z.B. eine zur betreffenden Gehäusehälfte und ggf. zu weiteren Trägerelementen umlaufende Dichtstruktur. Alternativ wird die Funktion des Verbindens der Teile und des Abdichtens der Teile gegeneinander durch getrennte Funktionselemente verwirklicht.

In einer besonders bevorzugten Ausgestaltung sind die beiden Gehäusehälften und das mindestens eine Trägerelement vor dem Zusammenklappen in einer Reihe oder über Eck angeordnet. Mit der Anzahl der Trägerelemente nimmt jedoch die Anzahl der Dichtungsschnittstellen zu, wodurch diese praktisch auf einige wenige beschränkt ist.

In einer bevorzugten Ausgestaltung ist der erfindungsgemäße Bioreaktor wie eine Flusszelle aufgebaut. In einer besonders bevorzugten Ausgestaltung ist hierzu zumindest eine der beiden Gehäusehälften mit einem, zwei oder ggf. mehreren fluidischen Anschlüssen versehen, die die fluidische Versorgung der in dem mindestens einen Zellkultursubstrat verankerten (adhärenten) Zellen mit gelösten Nährstoffen und Gasen und die fluidische Entsorgung der Stoffwechselprodukte der Zellen in Form einer Perfusion, einer ein- oder beidseitigen Superfusion oder einer Mischform hiervon über ein Kulturmedium ermöglichen. Dadurch lassen sich auch physikalische bzw. chemische Gradienten im Bioreaktor über das Zellkultursubstrat einstellen. Die fluidischen Anschlüsse befinden sich nur oben bzw. unten oder lateral, vorzugsweise nicht lateral im Bereich von fluiddichten Verbindungen.

In einer alternativen Ausgestaltung ist mindestens eine der beiden Gehäusehälften mit einer permeablen oder semipermeablen Membran oder einem porösen Bereich versehen, die jeweils zum Austausch von Fluiden durch Perfusion oder Osmose dienen.

Kernstück des erfindungsgemäßen Bioreaktors ist das mindestens eine Zellkultursubstrat, das sich vorzugsweise für eine dreidimensionale Kultivierung von Zellen in Form einer Anordnung aus Mikrocontainern, wie z.B. aus der DE 41 32 379 C2 oder der DE 10 2004 03 5267 B3, eignet. Alternativ ist das mindestens eine Zellkultursubstrat für eine zweidimensionale Kultur in Form eines planaren Substrats geeignet.

In einer bevorzugten Ausgestaltung ist das mindestens eine Zellkultursubstrat für einen verbesserten passiven oder aktiven Stofftransport perforiert. Grundsätzlich denkbar sind auch mikro- oder nanostrukturierte Substrate. Das Zellkultursubstrat kann implizit (statistisch) oder explizit (regulär) mikro- oder nanostrukturiert sein.

Für den erfindungsgemäßen Bioreaktor besteht bezüglich seiner Gestaltung eine große Anzahl an Möglichkeiten im Hinblick auf die Auswahl des Folienmaterials, dessen Form und Größe, die Anzahl der jeweils mit einem oder mehreren Zellkultursubstraten bestückten Trägerelemente, die Anordnung der oberen Gehäusehälfte, der unteren Gehäusehälfte und des Trägerelements oder der Trägerelemente, die Ausführung der Foliengelenke, der Mittel zum Austausch von Fluiden sowie Größe, Anzahl und Form der Zellkultursubstrate.

Das warmumformbare thermoplastische oder thermoelastische Folienmaterial für den Bioreaktor wird insbesondere nach den Gesichtspunkten Biokompatibilität, physikochemischer Beständigkeit, Transparenz und niedriger Eigenfluoreszenz ausgewählt.

Bevorzugt besteht der Bioreaktor aus Polymethylmethacrylat (PMMA), Polycarbonat (PC), Polyethylenterephthalat (PET), Polystyrol (PS), Polyimid (PI), Polyethylen (PE), Polypropylen (PP), Polyvinylfluorid (PVF), Polyvinylidenfluorid (PVDF), Polyvinylchlorid (PVC), Polyvinylidenchlorid (PVDF), Polyetherimid (PEI), Polyetheretherketon (PEEK), Polysulfon (PSU), Polyurethan (PU), Cycloolefincopolymer (COC) oder Cycloolefinpolymer (COP).

Alternativ besteht der Bioreaktor aus Polymilchsäure (PLA), aus Poly(ε-Caprolacton), aus einem Polyhydroxyalkanoat (PHA) oder einem anderen biologisch abbaubaren Polymer.

Die vorliegende Erfindung umfasst weiterhin eine Anordnung aus zusammenhängenden Einheiten von zwei oder mehreren bis hin zu einer Vielzahl von Bioreaktoren, die zu einem Stück zusammengefügt sind.

Ein erfindungsgemäßer Bioreaktor wird, bevorzugt einschließlich des Zellkultursubstrats, in Form einer Klappschachtel spritzgegossen oder als ein einziges Teil aus einem einzigen Stück Folienmaterial warmumgeformt, insbesondere thermogeformt, wobei die Gelenke und die flüssigkeitsdichten Verschlüsse mitangeformt werden.

Je nach Art und Dicke des Folienmaterials bzw. des Folienhalbzeugs für den Bioreaktor und der Ausformung des mindestens einen Trägerelements wird in einer bevorzugten Ausgestaltung das mindestens eine Zellkultursubstrat in dasselbe Halbzeug und im gleichen Formvorgang wie dem für die untere Gehäusehälfte und die obere Gehäusehälfte direkt in das über ein Foliengelenk mit diesen verbundene Trägerelement eingeformt.

In einer alternativen Ausgestaltung wird das mindestens eine Zellkultursubstrat in ein separates Halbzeug eingeformt und dieses dann zwischen die untere Gehäusehälfte und die obere Gehäusehälfte eingesetzt. Dabei bildet das mindestens eine Zellkultursubstrat mit den beiden Gehäusehälften eine flüssigkeitsdichte Verbindung.

In einer weiteren Ausgestaltung wird das gelenkig mittels eines weiteren Foliengelenks mit der unteren Gehäusehälfte, der oberen Gehäusehälfte oder ggf. einem weiteren Trägerelement verbundene Trägerelement als Rahmen ausgebildet, in den dann mindestens ein Zellkultursubstrat eingesetzt wird.

Ist das Trägerelement mit der unteren Gehäusehälfte oder der oberen Gehäusehälfte gelenkig verbunden, lässt sich dieses zur Weiterverarbeitung durch Schneiden oder Stanzen der Folie bzw. durch Reißen der Folie entlang einer Perforationslinie vom verbleibenden Bioreaktor abtrennen.

Der hier vorliegende Warmumformprozess umfasst auch das in der Regel abschließende so genannte *Trimmen,* d. h. das Heraustrennen des geformten Teils durch Stanzen, Laserschneiden, Wasserstrahlschneiden o.ä. aus dem verbleibenden Folienmaterial.

In einer besonderen Ausgestaltung lassen sich sowohl Außen- als auch Innenkonturen auf diese Art trenntechnisch erzeugen. Über angeformte und freigestanzte oder an ausgestanzten Öffnungen angeschraubte fluidische Anschlüsse in den beiden Gehäusehälften kann, ähnlich wie in der DE 20 2006 012 978 U1, eine Per- oder Superfusion des Zellkultursubstrats mit Kulturmediums oder eine Kombination der beiden Modi erfolgen.

Der erfindungsgemäße Bioreaktor und die erfindungsgemäße Anordnung sind aus wenigen Teilen, insbesondere aus einem einzigen Teil aufgebaut. Durch diesen einfachen Aufbau aus, wie für thermogeformte, vergleichsweise dünnwandige Produkte typisch, relativ kleinen Mengen, vorzugsweise aus nur einem einzigen Material sind der erfindungsgemä-ße Bioreaktor und die erfindungsgemäße Anordnung aus ökologischer und ökonomischer Sicht als Einwegteil geeignet.

Der erfindungsgemäße Bioreaktor und die erfindungsgemäße Anordnung ermöglichen eine sterile Kultivierung von Zellen oder dienen als Implantat in den menschlichen bzw. tierischen Körper.

Die Erfindung wird im Folgenden anhand von Ausführungsbeispielen und den Figuren näher erläutert. Es zeigen:
- Fig. 1: Perspektivische Darstellung eines Bioreaktors mit einem einzigen Trägerelement in (a) ausgeklappter Form, (b) teilweise zugeklappter Form und (c) vollständig zugeklappter Form.
- Fig. 2: Perspektivische Darstellung eines Bioreaktors mit drei Trägerelementen in ausgeklappter Form.

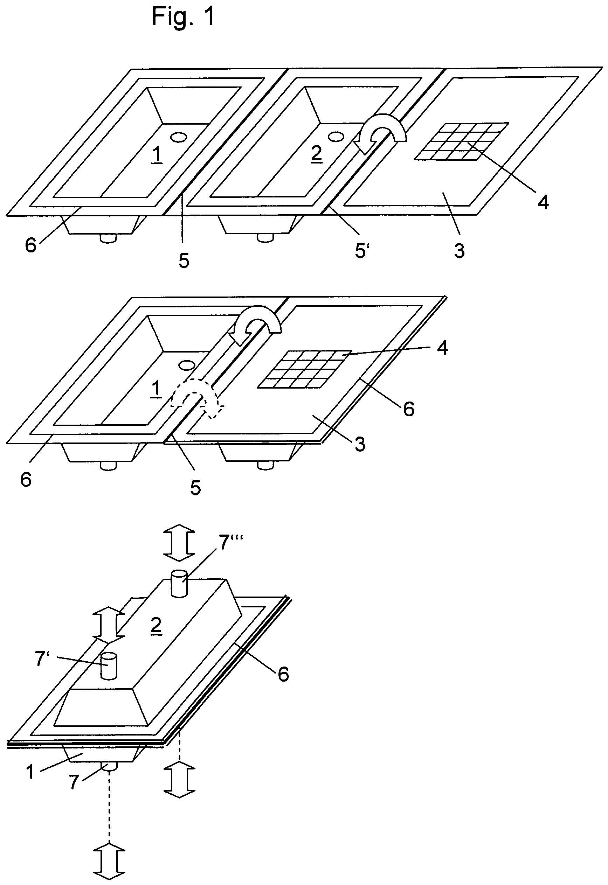

Fig. 1 zeigt in perspektivischer Darstellung einen erfindungsgemäßen Bioreaktor aus einer unteren Gehäusehälfte 1 und einer oberen Gehäusehälfte 2, die mittels eines Foliengelenks 5 jeweils an einer Seite miteinander verbunden sind, und einem Trägerelement 3, in das ein Zellkultursubstrat 4 eingefügt ist. Die gebogenen Pfeile geben die Richtung des Klappens und Dichtens an. Der gestrichelt gebogene Pfeil gibt eine alternative Weise für das Klappen an, nach der der Bioreaktor noch auf dem Kopf steht, was ein zusätzliches Umdrehen erfordert.

Das über ein weiteres Foliengelenk 5' mit der der oberen Gehäusehälfte 2 verbundene Trägerelement weist eine sowohl zur unteren Gehäusehälfte 1 als auch zur oberen Gehäusehälfte 2 passende, umlaufende Dichtstruktur 6 auf.

Die untere Gehäusehälfte 1 ist mit zwei fluidischen Anschlüssen 7, 7" (wobei der Anschluss 7" aufgrund der Perspektive nicht sichtbar ist) und die obere Gehäusehälfte 2 mit zwei fluidischen Anschlüssen 7', 7'" versehen. Die geraden Pfeile geben die Richtung des Durchströmens im Superfusions-, im
Perfusions- oder im gemischten Modus an.

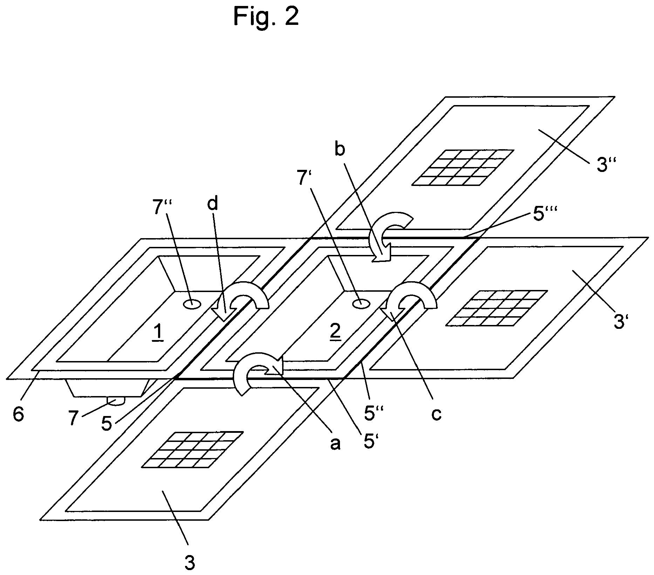

Fig. 2 zeigt in perspektivischer Darstellung einen erfindungsgemäßen Bioreaktors mit drei Trägerelementen 3, 3', 3'' und vier Foliengelenken 5, 5', 5", 5'''. Die Anordnung der Gehäusehälften 1, 2 und der Trägerelemente 3, 3', 3" wurde so gewählt, dass sich das Werkzeug möglichst einfach ausführen ließ. Die Reihenfolge des Zusammenklappens des Bioreaktors ist durch die gebogenen Pfeile a, b, c, d angegeben.

## Patentansprüche

1. Bioreaktor, bestehend aus
- einem Gehäuse aus einer unteren Gehäusehälfte (1) und einer oberen Gehäusehälfte (2), die mittels eines Foliengelenks (5) jeweils an einer Seite miteinander verbunden sind, wobei an mindestens einer Gehäusehälfte Mittel zum Austausch von Fluiden vorgesehen sind, und
- mindestens einem Trägerelement (3) für mindestens ein Zellkultursubstrat (4),
wobei der Bioreaktor aus einem warmumformbaren thermoplastischen oder thermoelastischen Folienmaterial besteht.

2. Bioreaktor nach Anspruch 1, wobei das mindestens eine Trägerelement (3) in die untere Gehäusehälfte (1) oder in die obere Gehäusehälfte (2) eingefügt ist.

3. Bioreaktor nach Anspruch 1, wobei das mindestens eine Trägerelement (3) zwischen die untere Gehäusehälfte (1) und die obere Gehäusehälfte (2) eingefügt ist.

4. Bioreaktor nach einem der Ansprüche 1 bis 3, wobei die untere Gehäusehälfte (1) und die obere Gehäusehälfte (2) fluiddicht zueinander angeordnet sind.

5. Bioreaktor nach Anspruch 4, wobei das mindestens eine Trägerelement (3) fluiddicht zu mindestens einer Gehäusehälfte angeordnet ist.

6. Bioreaktor nach einem der Ansprüche 3 bis 5, wobei das mindestens eine Trägerelement (3) an einer Seite mittels eines weiteren Foliengelenks (5') mit der unteren Gehäusehälfte (1) oder der oberen Gehäusehälfte (2) verbunden ist und über das Foliengelenk (5') zwischen die untere Gehäusehälfte (1) und die obere Gehäusehälfte (2) einklappbar ist.

7. Bioreaktor nach Anspruch 6 mit mindestens einem weiteren Trägerelement (3'), das mittels eines weiteren Foliengelenks (5") mit der unteren Gehäusehälfte (1), mit der oberen Gehäusehälfte (2) oder mit mindestens einem Trägerelement verbunden ist.

8. Bioreaktor nach einem der Ansprüche 1 bis 7, wobei mindestens eine der Gehäusehälften mit mindestens einem Anschluss (7) und/oder einer permeablen oder semipermeablen Membran oder einem porösen Bereich zum Austausch von Fluiden versehen ist.

9. Bioreaktor nach einem der Ansprüche 1 bis 8, wobei das mindestens eine Zellkultursubstrat (4) in das mindestens eine Trägerelement (3) eingeformt ist oder das mindestens eine Trägerelement (3) als Rahmen ausgebildet ist, in den das mindestens eine Zellkultursubstrat (4) eingesetzt ist.

10. Bioreaktor nach einem der Ansprüche 1 bis 9, wobei das mindestens eine Zellkultursubstrat (4) perforiert ist.

11. Anordnung aus mindestens zwei zusammenhängenden Bioreaktoren gemäß einem der Ansprüche 1 bis 11.

12. Verfahren zur Herstellung eines Bioreaktors nach einem der Ansprüche 1 bis 10 oder einer Anordnung nach Anspruch 11, wobei der Bioreaktor oder die Anordnung aus einem Stück Folienmaterial warmumgeformt wird.

13. Verfahren nach Anspruch 12, wobei das mindestens eine Zellkultursubstrat (4) in dasselbe Halbzeug und im gleichen Formvorgang wie für mindestens eine der Gehäusehälften in das über ein Foliengelenk (5) mit diesem verbundene mindestens eine Trägerelement (3) eingeformt wird oder das mindestens eine Zellkultursubstrat (4) in ein separates Halbzeug eingeformt wird und dieses dann zwischen die untere Gehäusehälfte (1) und die obere Gehäusehälfte (2) eingesetzt wird.

14. Verwendung eines Bioreaktors nach einem der Ansprüche 1 bis 10 oder einer Anordnung nach Anspruch 11 zur Kultivierung von Zellen oder als Implantat.

## Claims

1. Bioreactor, consisting of
- a housing comprised of a lower housing half (1) and an upper housing half (2), which are connected to one another by means of a film joint (5) in each case at one side, wherein means are provided at least at one housing half for the exchange of fluids, and
- at least one support element (3) for at least one cell culture substrate (4),
wherein the bioreactor consists of a thermoformable thermoplastic or thermoelastic film material.

2. Bioreactor according to claim 1, wherein the at least one support element (3) is inserted into the lower housing half (1) or into the upper housing half (2).

3. Bioreactor according to claim 1, wherein the at least one support element (3) is inserted between the lower housing half (1) and the upper housing half (2).

4. Bioreactor according to any one of claims 1 to 3, wherein the lower housing half (1) and the upper housing half (2) are arranged fluid-tight in relation to one another.

5. Bioreactor according to claim 4, wherein the at least one support element (3) is arranged fluid-tight in relation to at least one housing half.

6. Bioreactor according to any one of claims 3 to 5, wherein the at least one support element (3) is connected at one side by means of a further film joint (5') to the lower housing half (1) or the upper housing half (2), and can be folded by means of the film joint (5') between the lower housing half (1) and the upper housing half (2).

7. Bioreactor according to claim 6, with at least one further support element (3'), which is connected by means of a further film joint (5") to the lower housing half (1), to the upper housing half (2), or to at least one support element.

8. Bioreactor according to any one of claims 1 to 7, wherein at least one of the housing halves is provided with at least one connection (7) and/or a permeable or semi-permeable membrane or a porous region for the exchange of fluids.

9. Bioreactor according to any one of claims 1 to 8, wherein the at least one cell culture substrate (4) is moulded into the at least one support element (3), or the at least one support element (3) is configured as a frame, inserted into which is the at least one cell culture substrate (4).

10. Bioreactor according to any one of claims 1 to 9, wherein the at least one cell culture substrate (4) is perforated.

11. Arrangement of at least two connected bioreactors according to any one of claims 1 to 11.

12. Method for producing a bioreactor according to any one of claims 1 to 10, or an arrangement according to claim 11, wherein the bioreactor or the arrangement are thermoformed from one piece of film material.

13. Method according to claim 12, wherein the at least one cell culture substrate (4) is formed in the same semi-finished product and in the same moulding cycle as for at least one of the housing halves, into the at least one support element (3), connected to this housing half by means of a film joint (5), or the at least one cell culture substrate (4) is formed into a separate semi-finished product, and this is then inserted between the lower housing half (1) and the upper housing half (2).

14. Use of a bioreactor according to any one of claims 1 to 10 or of an arrangement according to claim 11 for the cultivation of cells or as an implant.

## Revendications

1. Bioréacteur composé de :
- un boîtier formé d'une moitié inférieure (1) et d'une moitié supérieure (2) reliées chaque fois d'un côté, par un film-charnière (5) au moins une moitié de boîtier comportant des moyens d'échange de fluides, et
- au moins un élément de support (3) pour au moins un substrat de culture cellulaire (4),
- le bioréacteur étant en un matériau en forme de film thermoplastique ou thermo-élastique déformables à chaud.

2. Bioréacteur selon la revendication 1,
**caractérisé en ce qu'**
au moins un élément de support (3) est inséré dans la moitié inférieure (1) ou dans la moitié supérieure (2).

3. Bioréacteur selon la revendication 1,
**caractérisé en ce qu'**
au moins un élément de support (3) est inséré entre la moitié inférieure (1) et la moitié supérieure (2).

4. Bioréacteur selon l'une des revendications 1 à 3,
**caractérisé en ce que**
la moitié inférieure (1) et la moitié supérieure (2) du boîtier sont associées de manière étanche aux fluides.

5. Bioréacteur selon la revendication 4,
**caractérisé en ce qu'**
au moins un élément de support (3) est associé d'une manière étanche aux fluides à au moins une moitié de boîtier.

6. Bioréacteur selon l'une des revendications 3 à 5,
**caractérisé en ce qu'**
au moins un élément de support (3) est relié sur un côté par un autre film-charnière (5') à la moitié inférieure (1) ou à la moitié supérieure (2) du boîtier et peut être rabattu par le film-charnière (5') entre la moitié inférieure (1) et la moitié supérieure (2) du boîtier.

7. Bioréacteur selon la revendication 6,
**caractérisé par**
au moins un autre élément de support (3') qui est relié par un autre film-charnière (5") à la moitié inférieure (1) du boîtier, à la moitié supérieure (2) du boîtier ou à au moins un élément de support.

8. Bioréacteur selon l'une des revendications 1 à 7,
**caractérisé en ce que**
pour l'échange de fluides au moins une des moitiés de boîtier est munie d'au moins un branchement (7) et/ou d'une membrane perméable ou semi-perméable ou d'une zone poreuse.

9. Bioréacteur selon l'une des revendications 1 à 8,
**caractérisé en ce qu'**
au moins un substrat de culture cellulaire (4) est formé dans au moins un élément de support (3) ou au moins un élément de support (3) est réalisé sous la forme de châssis recevant au moins un substrat de culture cellulaire (4).

10. Bioréacteur selon l'une des revendications 1 à 9,
**caractérisé en ce qu'**
au moins un substrat de culture cellulaire (4) est perforé.

11. Dispositif composé d'au moins deux bioréacteurs réunis selon l'une des revendications 1 à 11.

12. Procédé de réalisation d'un bioréacteur selon l'une des revendications 1 à 10 ou d'un dispositif selon la revendication 11, le bioréacteur ou le dispositif étant formé à chaud à partir d'une pièce de matière en forme de film.

13. Procédé selon la revendication 12,
**caractérisé en ce qu'**
au moins un substrat de culture cellulaire (4) est formé dans le même semi-produit et au cours de la même opération de formage, qu'au moins l'une des moitiés de boîtier dans un élément de support (3) relié à celle-ci par un film charnière (5) ou **en ce qu'**au moins un substrat de culture cellulaire (4) est formé dans un produit semi-fini séparé et celui-ci est ensuite placé entre la moitié inférieure (1) et la moitié supérieure (2) du boîtier.

14. Utilisation d'un bioréacteur selon l'une des revendications 1 à 10 ou d'un dispositif selon la revendication 11 pour cultiver des cellules ou comme implant.
